## Europäisches Patentamt

## European Patent Office

## Office européen des brevets

(11) Veröffentlichungsnummer: **0 036 669**
**B1**

(12) **EUROPÄISCHE PATENTSCHRIFT**

(45) Veröffentlichungstag der Patentschrift:
**14.12.83**

(21) Anmeldenummer: **81102225.0**

(22) Anmeldetag: **25.03.81**

(51) Int. Cl.³: **B 01 J 27/16,** B 01 J 35/10, C 07 C 29/04

(54) **Verfahren zur Herstellung eines Katalysators für die Hydratation von Olefinen.**

(30) Priorität: **26.03.80 DE 3011610**

(43) Veröffentlichungstag der Anmeldung:
**30.09.81 Patentblatt 81/39**

(45) Bekanntmachung des Hinweises auf die Patenterteilung:
**14.12.83 Patentblatt 83/50**

(84) Benannte Vertragsstaaten:
**BE DE FR GB IT NL**

(56) Entgegenhaltungen:
**EP - A - 0 018 022**
**EP - A - 0 023 071**
**DE - B - 1 156 772**
**US - A - 3 554 926**

(73) Patentinhaber: **CHEMISCHE WERKE HÜLS AG,**
**Postfach 1320, D-4370 Marl 1 (DE)**

(72) Erfinder: **Sommer, August, Dr., Birnenbruchstrasse 10,**
**D-4690 Herne 1 (DE)**
Erfinder: **Heitmann, Wilhelm, Dr., Bahnhofstrasse 7c,**
**D-4690 Herne 1 (DE)**
Erfinder: **Brücker, Rainer, Dr., Unterspredey 65,**
**D-4620 Castrop-Rauxel (DE)**

(74) Vertreter: **Steil, Hanna, Dipl.-Chem., RSP PATENTE -**
**PB 15 Postfach 1320, D-4370 Marl 1 (DE)**

## Verfahren zur Herstellung eines Katalysators für die Hydratation von Olefinen

Es ist bekannt, dass sich Olefine in der Gasphase bei erhöhten Drücken mit Wasserdampf zu Alkoholen umsetzen lassen. Besondere technische Bedeutung haben solche Verfahren bei der Herstellung von Ethylalkohol aus Ethylen und Isopropylalkohol aus Propylen erlangt. Die Synthese dieser Alkohole wird in Gegenwart von Katalysatoren durchgeführt, und zwar dient als Katalysator in aller Regel Phosphorsäure, die auf Trägern aufgebracht ist.

Bekannt sind Trägermaterialien entweder auf der Basis reiner Kieselsäure (z.B. Kieselgur oder Kieselgel) oder auf der Basis von Kieselsäure mit mehr oder weniger grossem Tonerdegehalt, wie z.B. calcinierte Diatomeenerde, deren Struktur durch Ton oder tonartige Stoffe zusammengehalten wird.

Bei den Trägern auf Basis reiner Kieselsäure ist die Festigkeit über längere Standzeiten problematisch. Die tonerdehaltigen Materialien zeichnen sich zwar durch eine bessere mechanische Festigkeit aus, sie haben jedoch bei zu hohem Tonerdegehalt den Nachteil, dass während der Reaktion das Aluminiumoxid durch die Einwirkung der Phosphorsäure herausgelöst wird.

In DE-C-1 156 772 wurde ein Verfahren beschrieben, einen tonerdehaltigen Träger für die bei der Olefinhydratation als Katalysator verwendete Phosphorsäure herzustellen, in dem geformte Kontaktkörper aus mineralischen Tonerdesilikaten derart mit Mineralsäure behandelt werden, dass der Aluminiumoxidgehalt vorzugsweise auf zwischen 1 und 5 Gew.-% abgesenkt wird. Dieses Material weist im allgemeinen sowohl die erforderliche mechanische Festigkeit auf als auch einen ausreichend niedrigen Rest-Aluminiumoxidgehalt zur Vermeidung des Herauslösens. Andererseits wurde beim Einsatz von handelsüblichen Kontaktkörpern aus mineralischen Rohstoffen für die Herstellung von Katalysatorträgern für die Hydratation von Olefinen beobachtet, dass ohne Vorauswahl des Rohstoffes stark unterschiedliche Katalysator-Aktivitäten gefunden werden.

Schliesslich war es gelungen, auch auf der Basis grobporiger Kieselgele Träger für Phosphorsäure mit hoher Hydratationsaktivität und ausreichender mechanischer Festigkeit zu entwickeln, z.B. gemäss den DE-A-2 625 705 und 2 719 055. Allerdings verbleibt als Nachteil dieser Träger auf der Basis amorpher Kieselsäure, dass bei längerem Aussetzen gegen die Bedingungen der Hydratationsreaktion die amorphe Kieselsäure teilweise zu Cristobalit und Quarz kristallisiert, was mit starker Verminderung der spezifischen Oberfläche und damit der katalytischen Aktivität, und zwar irreversibel, verbunden ist, sowie mit einer Abnahme der mechanischen Festigkeit.

Ein weiterer Nachteil aller bisher eingesetzten Hydratationskatalysatoren auf Basis Phosphorsäure, aufgebracht auf silikatischem Träger ist das langsame Abnehmen der Aktivität durch ausgetragene Phosphorsäure. Durch die inzwischen entwickelte kontinuierliche Einspritzung der ausgetragenen Phosphorsäuremenge gemäss DE-A-2 658 946 konnte zwar der laufende Aktivitätsverlust weitgehend vermieden und damit die Katalysator-Lebensdauer beträchtlich verlängert werden. Damit sind aber entsprechende Anforderungen an die Lebensdauer des Trägers zu stellen, so dass die Verwendung solcher Träger ausscheidet, bei denen unter Reaktionsbedingungen Kristallisationen unter Verminderung der katalytischen Aktivität auf irreversible Weise ablaufen und die mechanische Festigkeit im Laufe der Zeit abnimmt.

Gemäss DE-A-2 908 491 (EP-A-18 022) lässt sich aus Tonmineralien dann ein Träger für einen Hydratationskatalysator gleichbleibend hoher katalytischer Aktivität erhalten, wenn durch sorgfältige Auswahl des Rohstoffes Vorsorge dafür getroffen wird, dass das Material in hohem Masse aus Montmorillonit besteht, was dazu führt, dass nach Formung, Auslaugung und Tränkung die aktive Oberfläche, an der also die Hydratation des Olefins stattfinden kann, gross ist.

DE-A-2 908 491 betrifft ein Verfahren zur Herstellung eines Katalysators aus Tonmineralien für die Hydratation von Olefinen mit 2-3 C-Atomen zu den entsprechenden Alkoholen aus Phosphorsäure und Trägermaterial — sowie den danach hergestellten Katalysator — bei dem man einen im wesentlichen montmorillonithaltigen Ton, der mit nicht mehr als 3% Begleitmineralien, wie Quarz, Feldspat und Glimmer, verunreinigt ist und bis 0,5% $K_2O$ enthalten kann, zunächst in einer ersten Stufe mit Säure behandelt, bis er einen $Al_2O_3$-Gehalt von 13-18 Gew.-% aufweist und gegebenenfalls durch Zugabe von gefällter Tonerde den $Al_2O_3$-Gehalt auf 16-18 Gew.-% einstellt, wobei sich eine Oberfläche von 200-400 $m^2/g$, bevorzugt 240-300 $m^2/g$, ergibt, dann bei einem Gesamtwassergehalt von 20-35% durch Pressen formt, bei 500-800°C calciniert und anschliessend das geformte Trägermaterial in einer zweiten Stufe mit Säure bis auf einen $Al_2O_3$-Gehalt von 1-5 Gew.-%, bevorzugt 1-3 Gew.-%, behandelt, wobei sich eine Oberfläche von 150-250 $m^2/g$, bevorzugt 180-220 $m^2/g$, ergibt und den so erhaltenen Träger in bekannter Weise mit Phosphorsäure tränkt.

Anstelle von Montmorillonit kann dabei auch ein anderes Mineral der Montmorillonitgruppe eingesetzt werden, das kein Kalium, aber das Montmorillonit-Kristallgitter enthält.

Es ist dabei auch möglich, anstelle eines noch nicht säurebehandelten montmorillonithaltigen Tons eine bereits einmal säurebehandelte Bleicherde, hergestellt aus stark montmorillonithaltigem Ton, einzusetzen, wobei sich dann die erste Säurebehandlung erübrigt. Diese Bleicherde soll weniger als 0,1% $K_2O$ enthalten, das Gewichtsverhältnis ($Al_2O_3$ + $Fe_2O_3$) : $SiO_2$ soll 1 : 3,5 bis 1 : 4,5 betragen. Falls nötig, kann der $Al_2O_3$-Gehalt der Bleicherde durch Zugabe von gefällter Tonerde auf die erforderlichen 16-18 Gew.-% eingestellt werden.

Die so gezielt hergestellten Katalysatoren bzw. Katalysatorträger aus montmorillonithaltigem Ton zeigen gegenüber solchen, hergestellt aus geformten Kontaktkörpern auf Basis mineralischer Tonerdesilikate unterschiedlicher Provenienz, eine erhöhte Aktivität, d.h. es werden pro h und l Katalysatorschüttung etwa 105-110 g Ethanol bzw. ca. 300 g Isopropylalkohol gewonnen. Diese erhöhte Aktivität kann jedoch nur über einen längeren Zeitraum aufrechterhalten werden, wenn die ausgetragene Phosphorsäure, die bei Ethanol etwa 0,07 g pro h und l Katalysatorschüttung beträgt, bei Isopropylalkohol etwa 0,01 g pro h und l Katalysatorschüttung, durch kontinuierliche Zugabe der gleichen Säuremenge ausgeglichen wird.

DE-A-2 929 919 (EP-A-23 071) betrifft eine weitere Verbesserung des vorgenannten Verfahrens, bei der man den drei genannten Rohstoffen, also entweder dem montmorillonithaltigen Ton mit nicht mehr als 3% Begleitmineralien, wie Quarz, Feldspat und Glimmer und einem $K_2O$-Gehalt unter 0,5% oder einem anderen Mineral der Montmorillonitgruppe, das kein Kalium, aber das Montmorillonit-Kristallgitter enthält oder einer bereits säurebehandelten Bleicherde, hergestellt aus stark montmorillonithaltigem Ton vor dem Formen durch Pressen und Calcinieren bei 500-800°C 5-15 Gew.-%, bezogen auf die Gesamttrockensubstanz, an einem oder mehreren Oxiden von Metallen der VI. Nebengruppe des Periodensystems zugibt.

Mit dem Verfahren gemäss DE-A-2 929 919 werden insbesondere folgende Verbesserungen erzielt:

a) Anstieg der Kugeldruckfestigkeit des Katalysators

b) Reduzierung der Phosphorsäureaustragung unter Reaktionsbedingungen

c) Anstieg der Katalysatoraktivität auf etwa 130 g Ethanol und etwa 350 g Isopropylalkohol pro h und l Katalysatorschüttung.

Überraschenderweise wurde nun gefunden, dass künstlich hergestelltes Aluminiumsilikat als Träger für Hydratationskatalysatoren dem natürlichen Montmorillonit überlegen ist, wenn durch Wahl einer genügend hohen Konzentration an Aluminiumsalz und löslichem Kieselsäuresalz dafür gesorgt wird, dass sich bei der Fällung durch Vereinigen der Lösungen ein Gewichtsverhältnis $Al_2O_3 : SiO_2$ von 1 : 5 bis 1 : 7 im unlöslichen Aluminiumsilikat ergibt. Hierbei wird eine Art künstlicher Bleicherde erzeugt, dieser Begriff wurde z.B. von M. Nekritsch, Zeitschrift für anorganische Chemie 177 (1929) S. 86 verwendet. Die künstliche Bleicherde entspricht in der Zusammensetzung der natürlichen Bleicherde, also einem Montmorillonit nach der ersten Behandlungsstufe mit 20%iger Salzsäure. Allerdings kann diese künstliche Bleicherde bereits mit einer erheblich grösseren spezifischen Oberfläche hergestellt werden, als die im mineralischen Material nach einer Säurebehandlung vorliegt, nämlich 350-450 $m^2$/g gegenüber 200-400 $m^2$/g, insbesondere 240-300 $m^2$/g. Nach Formen, Calcinieren und der Säurebehandlung bei 100 bis 110°C, die der zweiten Säurebehandlung bei dem mineralischen Material entspricht, erniedrigt sich beim künstlich durch Fällung hergestellten Aluminiumsilikat die Oberfläche lediglich auf 280-380 $m^2$/g gegenüber 150-250 $m^2$/g, insbesondere 180-220 $m^2$/g bei dem mineralischen Material.

Gegenstand der Erfindung ist ein Verfahren zur Herstellung eines Katalysators für die Hydratation von Olefinen mit 2-3 C-Atomen zu den entsprechenden Alkoholen, aus Phosphorsäure und Trägermaterial aus Aluminiumsilikat, dadurch gekennzeichnet, dass ein künstliches, durch Fällen löslicher Salze von Aluminium und Kieselsäure gewonnenes Aluminiumsilikat, dessen Gewichtsverhältnis von $Al_2O_3 : SiO_2$ 1 : 5 bis 1 : 7 und dessen spezifische Oberfläche 350-450 $m^2$/g beträgt bei einem Gesamtwassergehalt von 20-35% durch Pressen geformt, bei 500-800°C calciniert und anschliessend einer Säurebehandlung unterzogen wird, wobei der $Al_2O_3$-Gehalt bis auf 1-5 Gew.-%, bevorzugt 1-3 Gew.-% und die Oberfläche auf 280-380 $m^2$/g abgesenkt und das Porenvolumen auf 1,1 bis 1,3 ml/g angehoben wird und man den so erhaltenen Träger in bekannter Weise mit Phosphorsäure tränkt.

Nach dem erfindungsgemässen Verfahren wird ein weiterer Aktivitätsanstieg des mit Phosphorsäure getränkten Trägers auf etwa 160 g Ethanol und 450 g Isopropylalkohol pro h und l Katalysatorschüttung erreicht.

Die Festigkeit des fertigen, mit Säure getränkten Katalysators liegt bei 7-9 kg/Körper und ist für das Beschicken der üblichen Reaktoren ausreichend.

Genau wie bei dem mineralischen Montmorillonit ist auch bei dem künstlichen eine Steigerung der Festigkeit auf etwa 11-13 kg/Körper zu erreichen, wenn auf die Gesamt-Trockensubstanz bezogen, 5-15 Gew.-% eines Oxides oder der Mischung mehrerer Oxide von Elementen der VI. Nebengruppe des Periodensystems vor der Formen durch Pressen und Calcinieren bei 500-800°C zugegeben werden.

Auch in diesem Fall kann die Austragung an Phosphorsäure etwa auf die Hälfte verringert werden, das heisst beim Ethanol von etwa 0,07 g pro h und l Katalysatorschüttung auf etwa 0,035 g pro h und l Katalysatorschüttung und beim Isopropylalkohol von etwa 0,01 g pro h und l Katalysatorschüttung auf etwa 0,005 g pro h und l Katalysatorschüttung.

Bei der bereits hohen Ausgangsaktivität des Trägers aus gefälltem Aluminiumsilikat ist durch das Zugeben von Oxiden von Elementen der VI. Nebengruppe des Periodensystems eine weitere Steigerung der Katalysatoraktivität nicht nachzuweisen.

*Beispiel 1*

Durch Zusammenmischung von je einer Lösung aus Aluminiumsulfat und Natriumsilikat in Wasser wurde ein künstliches Aluminiumsilikat als Niederschlag erhalten. Die Aluminiumsulfatlösung enthielt 16,6 kg $Al_2(SO_4)_3$ pro 100 l Wasser entsprechend 5,0 kg $Al_2O_3$ pro 100 l Wasser, die Natriumsilikatlösung enthielt 48,5 kg $Na_2SiO_3$ pro 100 l Wasser entsprechend 23,8 kg $SiO_2$ pro 100 l Wasser. Vereinigt wurden die Lösungen im Verhältnis 5 Teile Aluminiumsulfatlösung zu 6 Teile Natriumsilikatlösung, somit enthielt die Lösung nach der Vereinigung pro 100 l Wasser 7,5 kg $Al_2(SO_4)_3$ entsprechend 2,3 kg $Al_2O_3$ und 26,5 kg $Na_2SiO_3$ entsprechend 13,0 kg $SiO_2$. Der erhaltene Niederschlag enthielt nach dem Trocknen 13,2% $Al_2O_3$ und 86,8% $SiO_2$, das Gewichtsverhältnis $Al_2O_3 : SiO_2$ betrug also 1 : 6,5.

Dieses Material hatte eine spezifische Oberfläche von 387 $m^2$/g und ein Porenvolumen von 1,07 ml/g. Es wurde mit 43% Wasser auf die Trockensubstanz bezogen (das ist 30% Wasser auf die Gesamtmenge bezogen) angefeuchtet und zu Zylindern von 4 mm Durchmesser und 4 mm Höhe gepresst und danach durch 3 Stunden langes Erhitzen auf 600°C verfestigt.

Die so erhaltenen geformten Kontaktkörper wurden insgesamt zweimal für eine Stunde mit 20%iger Salzsäure bei 100-110°C behandelt und mit Wasser säurefrei gewaschen. Nach dem Trocknen bei etwa 110-120°C wurde in den Zylindern ein Aluminiumoxidgehalt von 1,6% gefunden, die spezifische Oberfläche betrug 352 m²/g, das Porenvolumen 1,18 ml/g. Die Formkörper wurden sodann mit 40 gew.%iger Phosphorsäure überflutet, die zwei Stunden einwirkte, danach wurde erneut bei etwa 110-120°C getrocknet. Diese so behandelten Zylinder hatten einen $H_3PO_4$-Gehalt von 38 Gew.-%.

Beim Einsatz dieses so fertiggestellten Hydratationskatalysators zur Synthese von Ethanol aus Ethylen und Wasser in der Gasphase unter üblichen Bedingungen konnte eine Katalysatorausbeute von 160 g Ethanol pro h und l Katalysatorschüttung erzielt werden.

Beim Einsatz dieses Materials zur Synthese von Isopropylalkohol aus Propylen und Wasser in der Gasphase konnte eine Katalysatorausbeute von 450 g Isopropylalkohol pro h und l Katalysatorschüttung erzielt werden.

Die Druckfestigkeit betrug 8 kg/Körper, die Säureaustragung während des Betriebes bei Ethanol 0,07 g pro h und l Katalysatorschüttung und bei Isopropylalkohol 0,01 g pro h und l Katalysatorschüttung.

*Beispiel 2*

Durch Zusammenmischung von je einer Lösung aus Aluminiumsulfat und Natriumsilikat in Wasser wurde ein künstliches Aluminiumsilikat als Niederschlag erhalten. Die Aluminiumsulfatlösung enthielt 16,6 kg $Al_2(SO_4)_3$ pro 100 l Wasser entsprechend 5,0 kg $Al_2O_3$ pro 100 l Wasser, die Natriumsilikatlösung enthielt 48,5 kg $Na_2SiO_3$ pro 100 l Wasser entsprechend 23,8 kg $SiO_2$ pro 100 l Wasser. Vereinigt wurden die Lösungen im Verhältnis 5 Teile Aluminiumsulfatlösung zu 6 Teile Natriumsilikatlösung, somit enthielt die Lösung nach der Vereinigung pro 100 l Wasser 7,5 kg $Al_2(SO_4)_3$ entsprechend 2,3 kg $Al_2O_3$ und 26,5 kg $Na_2SiO_3$ entsprechend 13,0 kg $SiO_2$. Der erhaltene Niederschlag enthielt nach dem Trocknen 13,2% $Al_2O_3$ und 86,8% $SiO_2$, das Gewichtsverhältnis $Al_2O_3$ : $SiO_2$ betrug also 1 : 6,5.

Dieses Material hatte eine spezifische Oberfläche von 387 m²/g und ein Porenvolumen von 1,07 ml/g. 100 Teile dieses Materials wurden mit 3 Teilen Chromoxid ($CrO_3$), 3 Teilen Molybdänoxid ($MoO_3$), 5 Teilen Wolframoxid ($WO_3$) gemischt, so dass die Mischung insgesamt 10% an Oxiden von Elementen der VI. Nebengruppe des Periodensystems enthielt. Nach Zugabe von 43% Wasser auf die Trockensubstanz bezogen (das ist 30% Wasser auf die Gesamtmenge bezogen) wurde es zu Zylindern von 4 mm Durchmesser und 4 mm Höhe gepresst und danach durch 3 Stunden langes Erhitzen auf 600°C verfestigt.

Die so erhaltenen geformten Kontaktkörper wurden insgesamt zweimal für eine Stunde mit 20%iger Salzsäure bei 100-110°C behandelt und mit Wasser säurefrei gewaschen. Nach dem Trocknen bei etwa 110-120°C wurde in den Zylindern ein Aluminiumoxidgehalt von 1,6% gefunden, die spezifische Oberfläche betrug 360 m²/g, das Porenvolumen 1,15 ml/g. Der Gehalt an Oxiden von Elementen der VI. Nebengruppe des Periodensystems hatte sich auf 12 Gew.-% erhöht.

Die Formkörper wurden sodann mit 40 gew.%iger Phosphorsäure überflutet, die zwei Stunden einwirkte, danach wurde erneut bei etwa 110-120°C getrocknet. Diese so behandelten Zylinder hatten einen $H_3PO_4$-Gehalt von 35 Gew.-%.

Beim Einsatz dieses so fertiggestellten Hydratationskatalysators zur Synthese von Ethanol aus Ethylen und Wasser in der Gasphase konnte eine Katalysatorausbeute von 160 g Ethanol pro h und l Katalysatorschüttung erzielt werden.

Beim Einsatz dieses Materials zur Synthese von Isopropylalkohol aus Propylen und Wasser in der Gasphase konnte eine Katalysatorausbeute von 450 g Isopropylalkohol pro h und l Katalysatorschüttung erzielt werden.

Die Druckfestigkeit betrug 12 kg/Körper, die Säureaustragung während des Betriebes bei Ethanol 0,035 g pro h und l Katalysatorschüttung und bei Isopropylalkohol 0,005 g pro h und l Katalysatorschüttung.

**Patentansprüche**

1. Verfahren zur Herstellung eines Katalysators für die Hydratation von Olefinen mit 2-3 C-Atomen zu den entsprechenden Alkoholen, aus Phosphorsäure und Trägermaterial aus Aluminiumsilikat, dadurch gekennzeichnet, dass ein künstliches, durch Fällen löslicher Salze von Aluminium und Kieselsäure gewonnenes Aluminiumsilikat, dessen Gewichtsverhältnis von $Al_2O_3$ : $SiO_2$ 1 : 5 bis 1 : 7 und dessen spezifische Oberfläche 350-450 m²/g beträgt bei einem Gesamtwassergehalt von 20-35% durch Pressen geformt, bei 500-800°C calciniert und anschliessend einer Säurebehandlung unterzogen wird, wobei der $Al_2O_3$-Gehalt bis auf 1-5 Gew.-%, bevorzugt 1-3 Gew.-%, und die Oberfläche auf 280-380 m²/g abgesenkt und das Porenvolumen auf 1,1 bis 1,3 ml/g angehoben wird und man den so erhaltenen Träger in bekannter Weise mit Phosphorsäure tränkt.

2. Verfahren zur Herstellung eines Katalysators für die Hydratation von Olefinen mit 2-3 C-Atomen nach Anspruch 1, dadurch gekennzeichnet, dass man vor der Formung des Aluminiumsilikatträgers 5-15 Gew.-%, bezogen auf die Gesamttrockensubstanz, von einem oder mehreren Oxiden von Metallen der VI. Nebengruppe des Periodensystems, zugibt.

3. Katalysator aus Aluminiumsilikat für die Hydratation von Olefinen mit 2-3 C-Atomen, hergestellt nach den Ansprüchen 1 und 2.

**Claims**

1. Process for the preparation of a catalyst for the hydration of olefines having 2-3 C atoms to give the corresponding alcohols, comprising phosphoric acid and carrier material composed of aluminium silicate, characterised in that a synthetic aluminium silicate which is obtained by precipitation of soluble salts of aluminium and silicic acid and has a weight ratio of $Al_2O_3 : SiO_2$ of 1 : 5 to 1 : 7 and a specific surface area of 350-450 $m^2/g$ is moulded by pressing at a total water content of 20-35%, calcined at 500-800°C and then subjected to treatment with an acid, the $Al_2O_3$ content being decreased to 1-5% by weight, preferably 1-3% by weight, the specific surface area being decreased to 280-380 $m^2/g$ and the pore volume being increased to 1.1-1.3 ml/g, and the carrier thus obtained is impregnated with phosphoric acid in a known manner.

2. Process for the preparation of a catalyst for the hydration of olefines having 2-3 C atoms according to claim 1, characterised in that 5-15% by weight, relative to the total dry substance, of one or more oxides of metals of sub-group VI of the periodic table is added before the aluminium silicate carrier is moulded.

3. Catalyst comprising aluminium silicate for the hydration of olefines having 2-3 C atoms, prepared according to claims 1 and 2.

**Revendications**

1. Procédé pour la fabrication d'un catalyseur pour l'hydratation d'oléfines à 2-3 atomes de carbone, en les alcools correspondants, à partir d'acide phosphorique et d'un matériau de support en silicate d'aluminium, procédé caractérisé en ce que l'on prépare un silicate d'aluminium artificiel, par précipitation de sels solubles d'aluminium et d'acide silicique, dont le rapport en poids $Al_2O_3/SiO_2$ est de 1:5 à 1:7 et dont la surface spécifique est de 350 à 450 $m^2/g$, avec une teneur en eau de 20 à 25%, on forme ce matériau par pressage en cylindres ou cubes, qu'on calcine à 500-800°C et on le soumet finalement à un traitement par un acide, de telle sorte que la teneur en $Al_2O_3$ soit de 1 à 5% en poids (et particulièrement de 1 à 3%) que la surface spécifique soit abaissée à 280-380 $m^2/g$ et que le volume de pores soit accru à 1,1 à 1,3 ml/g, le matériau de support ainsi obtenu étant finalement imprégné avec de l'acide phosphorique de la manière connue.

2. Procédé pour la fabrication d'un catalyseur pour l'hydratation d'oléfines à 2-3 atomes de carbone suivant la revendication 1, caractérisé en ce que, avant le formage des corps porteurs en silicate d'aluminium, on ajoute de 5 à 15% en poids, rapporté à la manière sèche totale, d'un ou plusieurs oxydes de métaux du groupe VI de la classification périodique des éléments.

3. Catalyseur pour l'hydratation d'oléfines à 2 à 3 atomes de C conforme au silicate d'aluminium artificiel obtenu conformément à l'une des revendications 1 et 2.